# EUROPEAN PATENT APPLICATION

(11) **EP 3 078 319 A1**
(43) Date of publication of application: **12.10.2016**
(21) Application number: 15800227.9
(22) Date of filing: 13.03.2015
(51) Int. Cl.: A61B 1/00, G02B 23/24

(54) **ENDOSCOPE SYSTEM**

(30) Priority: 29.05.2014 JP 2014111392
(71) Applicant: Olympus Corporation, Tokyo 192-8507 (JP)
(72) Inventor: SAITO Seiji, Hachioji-shi Tokyo 192-8507 (JP); OKANIWA Suguru, Hachioji-shi Tokyo 192-8507 (JP)
(74) Representative: Schicker, Silvia
(86) International application number: PCT/JP2015/057456
(87) International publication number: WO 2015/182215

(57) **Abstract**

An endoscope system 1 includes an endoscope 2 and a plurality of over-tubes 3A, 3B, and 3C provided with balloons 35A, 35B, and 35C to be expanded and contracted on distal end sides of tube insertion sections 31 A, 31 B, and 31 C having flexibility, in which outer diameters of the tube insertion sections 31A, 31B, and 31C are different from one another. A size relation of expansion time diameters Ar, Br, and Cr and a size relation of axial direction lengths Al, Bl, and Cl of the balloons 35A, 35B, and 35C provided in the tube insertion sections 31A, 31B, and 31C of the plurality of over-tubes 3A, 3B, and 3C coincide with a size relation of outer diameters Ao, Bo, and Co of the tube insertion sections 31 A, 31B, and 31C.

## Description

### Technical Field

The present invention relates to an endoscope system including an endoscope and an over-tube provided with a balloon on a distal end side of an insertion section tube into which an insertion section of the endoscope is inserted.

### Background Art

Endoscopes are widely used in a medical field and an industrial field.

With the endoscope for medical use, it is possible to perform observation of a test target part and, when necessary, curative treatment using a treatment instrument by inserting an elongated insertion section into a body cavity.

In the endoscope, a bending section for improving insertability into a body of a patient and making it possible to direct an observation optical system in a desired direction is provided. The insertion section of the endoscope is inserted toward a deep part in the body according to, for example, hand operation for bending the bending section and hand operation for twisting the insertion section.

However, when the insertion section of the endoscope is inserted into a deep digestive tract, for example, a small intestine, if the insertion section is simply pushed in, force is less easily transmitted to a distal end portion of the insertion section because of complicated bending of an intestinal canal. It is difficult to insert the insertion section into the deep part.

An endoscope over-tube is publicly known as an insertion supporting instrument for endoscope for inserting the insertion section into the body. By inserting the over-tube for endoscope into a body cavity together with, for example, the endoscope, it is possible to secure an insertion route of the endoscope into the body cavity. It is possible to easily perform insertion and removal of the endoscope afterwards.

For example, Japanese Patent Application Laid-Open Publication No. 2002-301019 describes an endoscope apparatus including an endoscope in which a balloon for endoscope is attached to a distal end portion outer circumferential section of an insertion section and a sliding tube (in an embodiment, described as an over-tube) in which a balloon for tube fixing is attached to a distal end portion outer circumferential section.

In the endoscope apparatus, the insertion section of the endoscope is inserted through the sliding tube, air in the balloon for endoscope provided in the insertion section and the balloon for tube fixing provided in the sliding tube is let out to deflate the balloons, and the insertion section is inserted into a body cavity.

When distal ends of the insertion section and the sliding tube are inserted to, for example, a descending portion of a duodenum, the balloon for tube fixing attached to the distal end of the sliding tube is inflated to fix the sliding tube to an intestinal canal. In this state, the insertion section is straightened as much as possible, the sliding tube is retained, and only the insertion section is inserted into a deep part.

Subsequently, after the insertion section is inserted a predetermined distance, air is supplied into the balloon for endoscope to inflate and fix the balloon to the intestinal canal.

Subsequently, the air in the balloon for tube fixing is released to deflate the balloon for tube fixing and the sliding tube is inserted into the deep part along the insertion section. A distal end portion of the sliding tube is inserted to near the distal end of the insertion section. The balloon for tube fixing is inflated again to fix the sliding tube to an intestinal wall.

Subsequently, a manipulation for, after deflating the balloon for endoscope, retaining the sliding tube as explained above, and inserting only the insertion section into the deep part the predetermined distance to inflate the balloon and fix the sliding tube to the intestinal canal, a manipulation for deflating the balloon for tube fixing, inserting the sliding tube into the deep part along the insertion section, and inflating the balloon for tube fixing to fix the sliding tube to the intestinal canal, and the like are repeatedly performed to advance a distal end portion of the insertion section to a deep part of a small intestine.

Note that a manipulation for causing a bending section of the insertion section to perform a bending action to fix an angled insertion section distal end portion to the intestinal canal instead of inflating the balloon for endoscope provided at the distal end portion of the insertion section to fix the insertion section to the intestinal canal, a manipulation for inserting the sliding tube into the deep part along the insertion section, a manipulation for inflating the balloon for tube fixing to fix the sliding tube to the intestinal canal, and the like may be repeated to advance the distal end portion of the insertion section to the deep part of the small intestine.

There are sliding tubes of various specifications. The sliding tubes are properly used according to purposes of use and endoscopes combined with the sliding tubes.

However, for example, in the sliding tube of specifications in which a balloon is provided at a distal end portion, irrespective of sizes of outer diameters of tube insertion sections, sizes of balloons, lengths of the balloons, and total lengths of the tube insertion sections are the same. In other words, in the sliding tubes of the same specifications, the balloons and the tube insertion sections are standardized among models in which the outer diameters of the tube insertion sections are different. Therefore, in over-tubes in which the balloons are provided at the distal end portions of the tube insertion sections, convenience of use changes because of the difference of the outer diameters of the tube insertion sections. It was difficult to obtain optimum specifications corresponding to purposes of use.

The present invention has been devised in view of the circumstances and an object of the present invention is to provide an endoscope system of optimum specifications excellent in convenience of use by selecting an over-tube including a tube insertion section provided with a balloon corresponding to a purpose of use and combining the over-tube with an endoscope.

### Disclosure of Invention

### Means for Solving the Problem

An endoscope system in an aspect of the present invention includes: an endoscope; and a plurality of over-tubes provided with balloons to be expanded and contracted on distal end sides of tube insertion sections having flexibility, in which at least outer diameters of the tube insertion sections are different from one another. A size relation of expansion time diameters and a size relation of axial direction lengths of the balloons provided on the distal end sides of the tube insertion sections of the plurality of over-tubes are set to coincide with a size relation of outer diameters of the tube insertion sections.

### Brief Description of the Drawings

Fig. 1 is a diagram for explaining an endoscope system including an endoscope and an over-tube;
Fig. 2 is a diagram for explaining a plurality of kinds of over-tubes provided with balloons on distal end sides of tube insertion sections;
Fig. 3A is a diagram for explaining action of a first over-tube, which is one of the plurality of over-tubes;
Fig. 3B is a diagram for explaining the action of the first over-tube, which is one of the plurality of over-tubes;
Fig. 3C is a diagram for explaining the action of the first over-tube, which is one of the plurality of over-tubes;
Fig. 3D is a diagram for explaining the action of the first over-tube, which is one of the plurality of over-tubes;
Fig. 3E is a diagram for explaining the action of the first over-tube, which is one of the plurality of over-tubes;
Fig. 3F is a diagram for explaining the action of the first over-tube, which is one of the plurality of over-tubes;
Fig. 3G is a diagram for explaining the action of the first over-tube, which is one of the plurality of over-tubes;
Fig. 4A is a diagram for explaining action of a second over-tube, which is one of the plurality of over-tubes;
Fig. 4B is a diagram for explaining the action of the second over-tube, which is one of the plurality of over-tubes;
Fig. 4C is a diagram for explaining the action of the second over-tube, which is one of the plurality of over-tubes;
Fig. 4D is a diagram for explaining the action of the second over-tube, which is one of the plurality of over-tubes; and
Fig. 4E is a diagram for explaining the action of the second over-tube, which is one of the plurality of over-tubes.

### Best Mode for Carrying Out the Invention

An embodiment of the present invention is explained below with reference to the drawings.

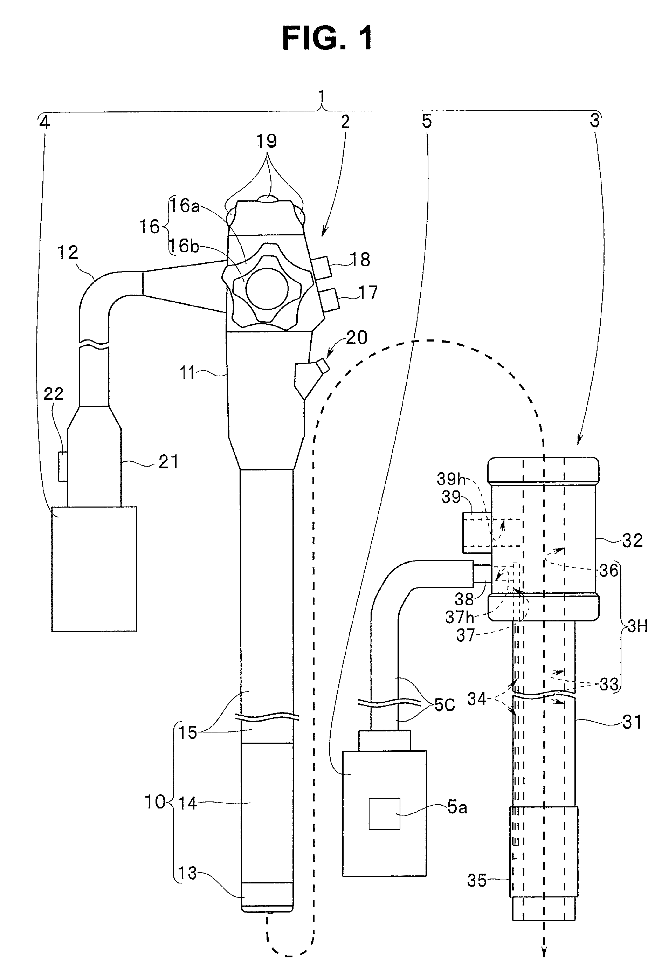

As shown in Fig. 1, an endoscope system 1 mainly includes an electronic endoscope (hereinafter referred to as endoscope) 2, an over-tube 3, a light source apparatus 4, an air feeding apparatus 5, a video processor (not shown in the figure), and a display apparatus (not shown in the figure). The endoscope 2 includes an insertion section 10, an operation section 11, and a universal cord 12. The insertion section 10 includes a distal end portion 13, a bending section 14, and a flexible tube section 15 in order from a distal end. An image pickup apparatus (not shown in the figure) and the like are incorporated in the distal end portion 13.

Note that specifications of the endoscope 2 are different depending on a diameter dimension and length of the insertion section 10, a configuration of the bending section 14, or the like.

The operation section 11 is provided on a proximal end side of the flexible tube section 15. A bending-section operation apparatus 16, an air/water feeding button 17, a suction switch 18, various remote switches 19, a treatment-instrument insertion port 20, and the like are provided in the operation section 11.

The bending-section operation apparatus 16 is, for example, a knob for causing the bending section 14 to perform a bending action in an up-down direction or a left-right direction. A knob for up and down 16a and a knob for left and right 16b are provided. As the various remote switches 19, a switch for performing freeze operation and the like, a switch for performing release operation and the like, and the like are provided.

An endoscope connector 21 detachably attachable to the light source apparatus 4 is proved at an extension end of the universal cord 12. A cable connecting section 22, to which a not-shown electric cable is detachably attachable, is provided in the endoscope connector 21. A processor connector detachably attachable to a video processor (not shown in the figure) is provided at an extension end of the electric cable.

The light source apparatus 4 includes, for example, a light source that supplies illumination light to the endoscope 2. The video processor processes an endoscopic image signal, which is photoelectrically converted by the image pickup apparatus provided in the insertion section 10 of the endoscope 2, into a video signal determined in advance and outputs the processed video signal to the display apparatus.

The over-tube 3 includes a tube insertion section 31 and a grasping section 32 configured in a cylindrical shape from a rigid member. A balloon 35 is provided at a distal end portion of the tube insertion section 31. The over-tube 3 is disposable.

The tube insertion section 31 is, for example, a multi-lumen tube made of silicone having flexibility. An endoscope-insertion-section passage (hereinafter referred to as insertion-section through-hole) 33 and a fluid conduit 34 are provided.

The balloon 35 is made of, for example, silicone that has elasticity and is stretchable.

The insertion-section through-hole 33 is an axial-direction through-hole. The insertion section 10 of the endoscope 2 is inserted through the insertion-section through-hole 33. An inner surface of the insertion-section through-hole 33 is applied with a hydrophilic lubrication coating treatment.

The fluid conduit 34 is a channel for supplying fluid for inflating the balloon 35 or discharging the fluid in the balloon 35.

A distal end opening of the insertion-section through-hole 33 is formed on a distal end face of the tube insertion section 31. A proximal end opening of the insertion-section through-hole 33 and a proximal end opening of the fluid conduit 34 are formed on a proximal end face of the tube insertion section 31. A distal end opening of the fluid conduit 34 is formed on a side surface of the tube insertion section 31 and within a balloon attachment range in which the balloon 35 is attached.

The grasping section 32 is, for example, bonded and fixed to a proximal end portion of the tube insertion section 31. A guide hole 36 and a fluid hole 37 are formed in the grasping section 32. The guide hole 36 is an axial-direction through-hole and communicates with the insertion-section through-hole 33 to configure a tube introducing hole 3H through which the insertion section 10 of the endoscope 2 is inserted.

The fluid hole 37 includes a distal end side opening disposed to be opposed to a proximal end side opening of the fluid conduit 34 and is set to depth determined in advance. Reference sign 37h denotes a first communication hole, which communicates with the fluid hole 37.

The fluid hole 37 is opened on a side surface of the grasping section 32. A fluid-tube connection pipe sleeve 38 is provided in the opening. An extension end of a fluid tube 5C extended from the air feeding apparatus 5, which is a fluid supply apparatus, is detachably coupled to the fluid-tube connection pipe sleeve 38.

In a state in which the fluid tube 5C is coupled to the fluid-tube connection pipe sleeve 38, when, for example, air is supplied from the air feeding apparatus 5, the air is supplied into the balloon 35 through the fluid tube 5C, the fluid-tube connection pipe sleeve 38, the first communication hole 37h, the fluid hole 37, and the fluid conduit 34. As a result, the balloon 35 gradually inflates.

Note that the fluid in the balloon 35 is discharged from the balloon through a route opposite to the route of the supply.

The supply or the discharge of the fluid is performed by a remote controller switch or a footswitch not shown in the figure.

Reference numeral 39 denotes a lubrication-liquid supply port, which includes a second communication hole 39h communicating with the guide hole 36. Sterilized water is fed into to guide hole 36 from the lubrication-liquid supply port 39 through the second communication hole 39h to wet a coating of the insertion-section through-hole 33, whereby a lubricity between the insertion section 10 and the insertion-section through-hole 33 is improved. The insertion section 10 can smoothly move in the insertion-section through-hole 33.

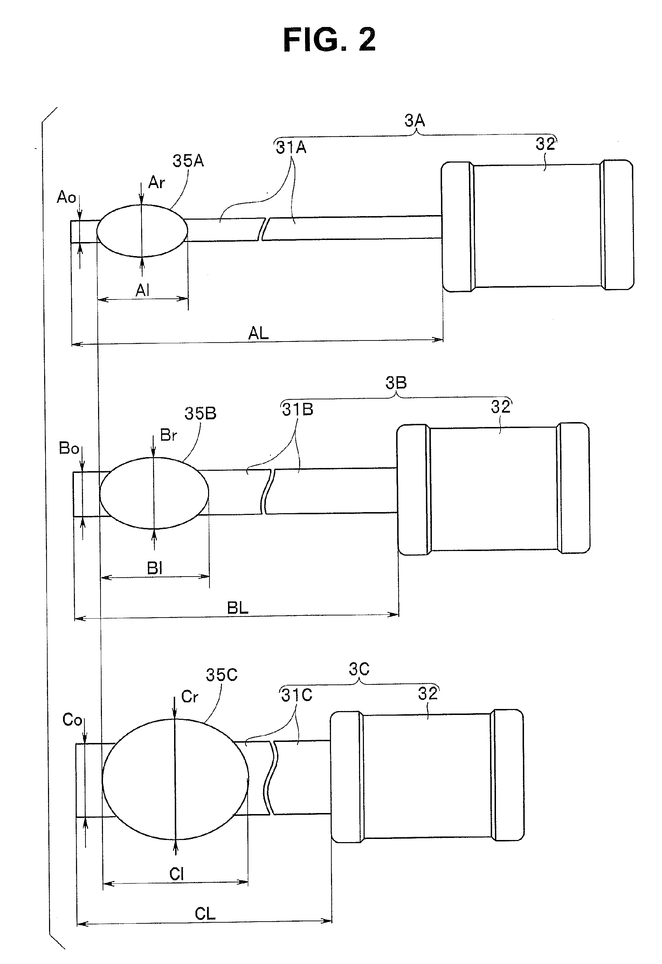

In the present embodiment, in the endoscope system 1 shown in Fig. 1, a plurality of over-tubes 3A, 3B, and 3C shown in Fig. 2 are prepared in advance rather than one over-tube 3.

The plurality of over-tubes 3A, 3B, and 3C are selectively used according to a purpose of use and specifications of the endoscope 2.

In the present embodiment, the first over-tube (hereinafter abbreviated as first tube) 3A is, for example, an over-tube for a small intestine, the second over-tube (hereinafter abbreviated as second tube) 3B is, for example, an over-tube for a large intestine, and the third over-tube 3C (hereinafter abbreviated as third tube) is, for example, an over-tube for an endoscope used with a hood attached.

An outer diameter of a tube insertion section 31A of the first tube 3A is Ao, an outer diameter of a tube insertion section 31B of the second tube 3B is Bo, and an outer diameter of a tube insertion section 31C of the third tube 3C is Co. The outer diameters Ao, Bo, and Co of the tube insertion sections 31A, 31B, and 31C are larger in the order of Ao, Bo, and Co.

Note that an inner diameter of the first tube insertion section 31A is set to a diameter larger than an outer diameter of an insertion section of an endoscope for a small intestine by a dimension determined in advance and is Ao. An inner diameter of the second tube insertion section 31B is set to a diameter larger than an outer diameter of an insertion section of an endoscope for a large intestine by a dimension determined in advance.

On the other hand, an inner diameter of the tube insertion section 31C of the third tube 3C is set to a diameter larger than an outer diameter of a hood attached to a distal end portion of an insertion section of an endoscope by a dimension determined in advance.

A first balloon 35A attached to the tube insertion section 31A of the first tube 3A has a balloon expansion time diameter Ar and a balloon longitudinal axial direction length Al. The diameter Ar and the length Al of the first balloon 35A are set taking into account a fixing force (referred to as fixing strength as well) to a small intestinal wall, a fluid supply time or a fluid discharge time, and the like.

A second balloon 35B attached to the tube insertion section 31B of the second tube 3B has a balloon expansion time diameter Br and a balloon longitudinal axial direction length Bl. The diameter Br and the length Bl of the second balloon 35B are set taking into account a fixing force to a large intestinal wall, an interval of folds, length of a bending section, and the like.

A third balloon 35C attached to the tube insertion section 31C of the third tube 3C has a balloon expansion time diameter Cr and a balloon longitudinal axial direction length Cl. The diameter Cr and the length Cl of the third balloon 35C are set taking into account a fixing force to the large intestinal wall, an interval of folds, length of a bending section, and the like as in the second tube 3B.

That is, in the over-tubes 3A, 3B, and 3C, a size relation of the diameters Ar, Br, and Cr at the balloon expansion time and a size relation of the lengths Al, Bl, and Cl in the balloon longitudinal axial direction are the same as a size relation of the outer diameters Ao, Bo, and Co of the tube insertion sections 31. In other words, the size relation of the expansion time diameters Ar, Br, and Cr and the size relation of the axial direction lengths Al, Bl, and Cl of the first balloons 35A, 35B, and 35C attached to the tube insertion sections 31A, 31B, and 31C of the plurality of tubes 3A, 3B, and 3C are set to coincide with the size relation of the outer diameters Ao, Bo, and Co of the tube insertion sections 31A, 31B, and 31C.

An effective length (hereinafter referred to as length) of the tube insertion section 31A of the first tube 3A is AL, length of the tube insertion section 31B of the second tube 3B is BL, and length of the tube insertion section 31C of the third tube 3C is CL. The tube insertion section lengths are shorter in order of AL, BL, and CL.

That is, a size relation of the lengths Al, BL, and CL of the tube insertion sections 31A, 31B, and 31C of the plurality of tubes 3A, 3B, and 3C is set to be opposite to the size relation of the outer diameters Ao, Bo, and Co of the tube insertion sections 31A, 31B, and 31C.

Action of the endoscope system 1 including the plurality of tubes 3A, 3B, and 3C is explained.

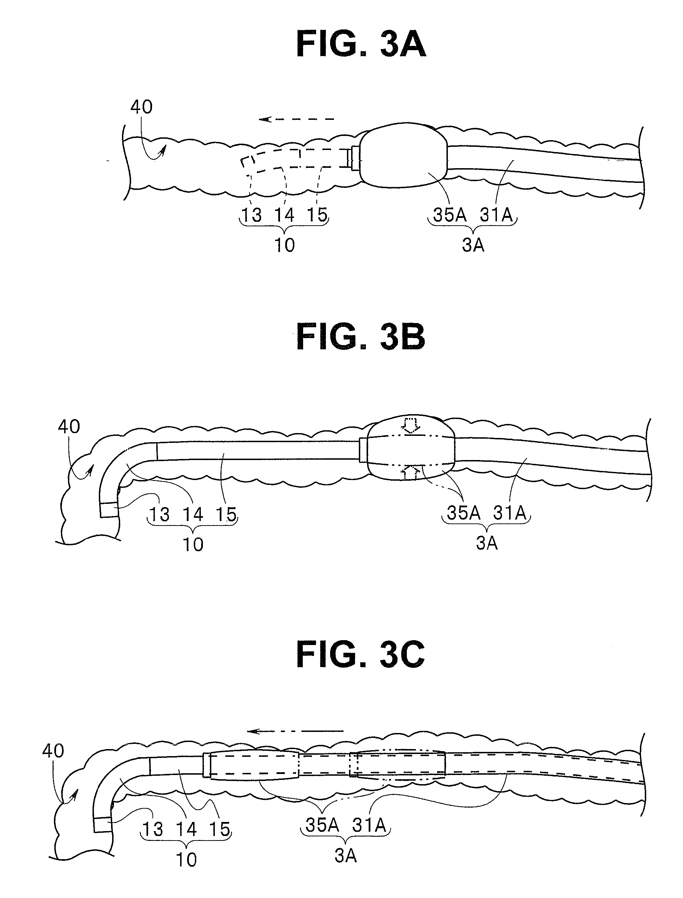

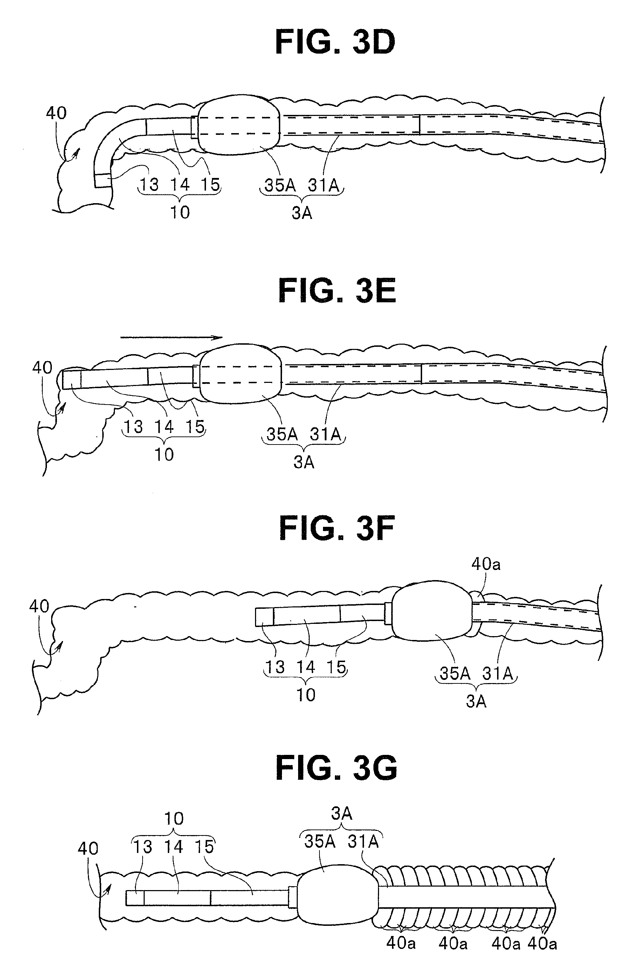

First, an example of action of the first tube 3A is explained with reference to Fig. 3.

When inserting the endoscope into a small intestine and performing a test or treatment, a surgeon combines the first tube 3A with the endoscope 2 for a small intestine test. In the small intestine test, the surgeon performs a manipulation for reducing an intestinal canal, introduces a distal end portion of the insertion section 10 into a deep part of the small intestine, and performs the test or the like.

At this time, first, the surgeon inserts and arranges the insertion section 10 of the endoscope 2 through the tube introducing hole 3H of the first tube 3A in a state in which the first balloon 35A is deflated. In this state, the surgeon inserts the insertion section 10 and the first tube 3A toward the small intestine.

When determining with an image displayed on the display apparatus that distal ends of the insertion section 10 and the first tube insertion section 31 A have reached a desired part of the small intestine, the surgeon once stops the inserting manipulation. Then, the surgeon operates, for example, the footswitch.

A control section 5a of the air feeding apparatus 5 starts supply of air and detection of pressure in the balloon 35 simultaneously with receiving an instruction signal from the footswitch. When detecting that the pressure in the balloon 35 has reached pressure determined in advance, the control section 5a determines that the first balloon 35A has reached a predetermined expanded state and stops the supply of the air.

At this point, an outer surface of the first balloon 35A in the expanded state determined in advance as shown in Fig. 3A is firmly attached and fixed to a wall surface of a small intestine 40. The first tube insertion section 31A is retained in the small intestine 40 at a fixing strength determined in advance.

Subsequently, in a state in which the first tube insertion section 31A is fixed to the small intestine 40, the surgeon inserts only the insertion section 10 toward a deep part of the small intestine 40 as indicated by a broken line arrow shown in Fig. 3A. After inserting the insertion section 10 by a distance determined in advance, the surgeon operates the bending-section operation apparatus 16 as appropriate while observing an endoscopic image displayed on the display apparatus and bends the bending section 14.

The intestinal canal is grasped by an insertion-section distal end portion 10a including the bending section 14 in a bent state as shown in Fig. 3B.

Subsequently, in a state in which the insertion-section distal end portion 10a grasps the intestinal canal, the surgeon operates the footswitch and outputs, to the control section 5a, an instruction signal for discharging the air in the first balloon 35A. Then, the first balloon 35A in the expanded state is contracted as indicated by an arrow in Fig. 3B.

When determining that the first balloon 35A has been contracted by the control section 5a, the surgeon advances the first tube insertion section 31A toward an insertion section distal end side along the insertion section 10 as indicated by an alternate long and two short dashes line in Fig. 3C.

After advancing the insertion section 10 by a distance determined in advance, the surgeon stops the advance.

Subsequently, the surgeon operates the footswitch again and supplies the air into the first balloon 35A. As a result, as shown in Fig. 3D, the first balloon 35A changes to the expanded state again and the first tube insertion section 31 A is fixed in a position in the small intestine 40 to which the first tube insertion section 31A is advanced.

Subsequently, in a state in which the first tube insertion section 31A is fixed to the small intestine 40, the surgeon operates the bending-section operation apparatus 16 as appropriate and releases a bent state of the bending section 14 as shown in Fig. 3E. The surgeon pulls back the first tube insertion section 31A and the insertion section 10 by a distance determined in advance as indicated by a slid line arrow.

Since the first tube 3 A and the insertion section 10 are pulled back, one folded section 40a is formed, the intestinal canal is folded, and the small intestine 40 is slightly reduced as shown in Fig. 3F.

Subsequently, the surgeon repeatedly performs hand operation for inserting only the insertion section 10 toward the deep part of the small intestine 40 as shown in Fig. 3A, hand operation for bending the bending section 14 and grasping the intestinal canal with the insertion-section distal end portion 10a as shown in Fig. 3B, hand operation for advancing the first tube insertion section 31A to the insertion section distal end side along the insertion section 10 as shown in Fig. 3C, hand operation for fixing the advanced first tube insertion section 31 A to the small intestine 40 as shown in Fig. 3D, hand operation for releasing the bent state as shown in Fig. 3E, and hand operation for pulling back the first tube insertion section 31A and the insertion section 10 and forming a new folded section 40a.

As a result, it is possible to form a plurality of folded sections 40a, 40a, ..., and 40a, reduce the intestinal canal, and insert the distal end portion 13 of the insertion section 10 to the deep part of the small intestine 40 as shown in Fig. 3G.

In this way, when the manipulation for inserting the insertion section 10 of the endoscope 2 to the deep part of the small intestine is performed, the first tube 3A in which the first balloon 35A is provided in the first tube insertion section 31A is selected. As a result, it is possible to expand and contract the first balloon 35A within a time determined in advance. Further, it is possible to firmly attach the first balloon 35A to an intestinal wall with a desired fixing force in a state in which the first balloon 35A is expanded.

As a result, it is possible to surely and smoothly perform, in a short time, a manipulation for forming a large number of folded sections and reducing the intestinal canal and perform a small intestine test and or like.

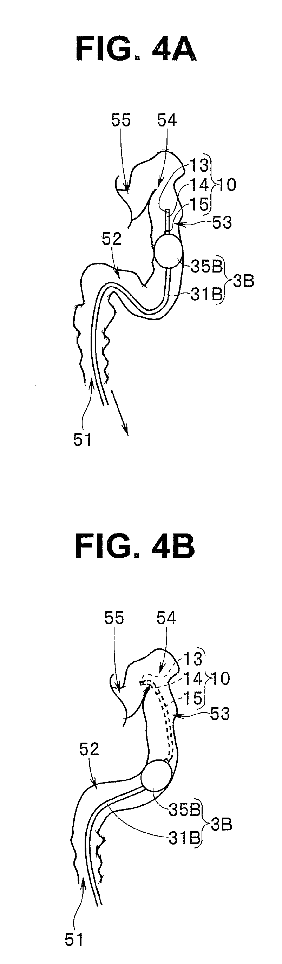

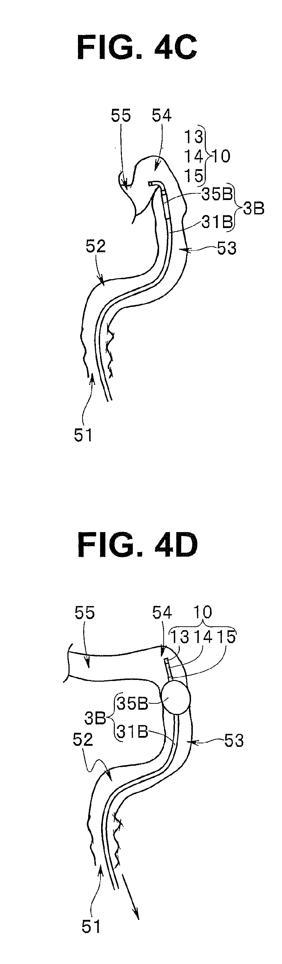

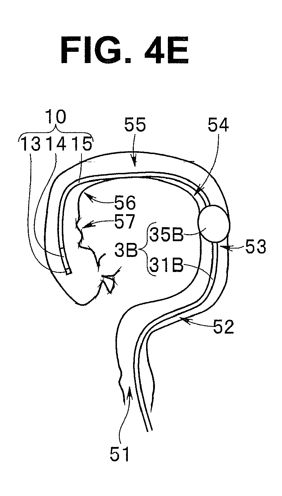

An example of use of the second tube 3B is explained with reference to Fig. 4.

When inserting the endoscope into a large intestine and perform a test or treatment, the surgeon combines the second tube 3B with the endoscope 2 for a large intestine test. In the large intestine test, the surgeon prevents extension of an intestinal canal while straightening the large intestine, introduces the distal end portion of the insertion section 10 to a deep part of the large intestine, and performs the test or the like.

At this time, first, the surgeon inserts and arranges the insertion section 10 of the endoscope 2 through the tube introducing hole 3H of the second tube 3B in a state in which the second balloon 35B is deflated. In this state, the surgeon inserts the insertion section 10 and the second tube insertion section 31B from an anus (see reference numeral 51 in Fig. 4) toward a large intestine deep part.

The surgeon performs hand operation such as bending operation or twisting operation while checking an endoscopic image displayed on the display apparatus and causes the insertion section 10 and the second tube insertion section 31B to pass through a sigmoid colon (see reference numeral 52 in Fig. 4). When determining that distal ends of the insertion section 10 and the second tube insertion section 31B have reached a desired part of a descending colon (see reference numeral 53 in Fig. 4), the surgeon stops the inserting manipulation and operates the footswitch.

Then, the control section 5a of the air feeding apparatus 5 starts supply of air into the second balloon 35B and inflates the balloon 35B. When determining that the second balloon 35B has reached a predetermined expanded state, the control section 5a stops the supply of the air. At this point, an outer surface of the second balloon 35B in the expanded state is firmly attached and fixed to a wall surface of a large intestine 50 as shown in Fig. 4A. The second tube insertion section 31B is retained on the large intestine 50 at a fixing strength determined in advance.

The surgeon pulls back the second tube insertion section 31B, in which the second balloon 35B is fixed to the large intestine 50, to a hand side by a distance determined in advance as indicated by a solid line arrow in Fig. 4A.

As a result, a section from the anus 51 to the sigmoid colon 52 and the descending colon 53 is substantially straightened and shortened as shown in Fig. 4B.

Subsequently, in a state in which the second balloon 35B is fixed to the large intestine 50, the surgeon inserts only the insertion section 10 toward a direction of a bending section of a splenic flexure 54 as indicated by a broken line in Fig. 4B.

When confirming that the distal end portion 13 of the insertion section 10 has reached a vicinity of the bending section of the splenic flexure 54, the surgeon performs hand operation such as bending operation or twisting operation. The surgeon hooks the insertion-section distal end portion 10a on the bending section of the splenic flexure 54 while bending the bending section 14 according to a bending shape of the splenic flexure 54.

Subsequently, in a state in which the insertion-section distal end portion 10a is hooked by the bending section of the splenic flexure 54, the surgeon operates the footswitch and contracts the second balloon 35B in the expanded state. Subsequently, the surgeon advances the second tube insertion section 31B toward the distal end portion 13 side of the insertion section 10 along the insertion section 10.

After advancing the insertion section 10 an amount determined in advance as shown in Fig. 4C, the surgeon once stops the operation. Subsequently, the surgeon operates the footswitch, changes the second balloon 35B to the expanded state again, and fixes the second tube insertion section 31B to the vicinity of the bending section of the splenic flexure 54.

In the fixed state explained above, the surgeon operates the bending-section operation apparatus 16 as appropriate and releases the insertion-section distal end portion 10a hooked by the bending section. Subsequently, the surgeon pulls back the second tube insertion section 31 B and the insertion section 10 as indicated by a solid line arrow. As a result, the bending section of the splenic flexure 54 is changed to a smooth bent shape and a transverse colon 55 is straightened as shown in Fig. 4E.

Subsequently, in a fixed state explained above, the surgeon guides only the insertion section 10 into the straightened transverse colon 55, guides the insertion section 10 to the transverse colon 55, the splenic flexure 56, and an ascending colon 57 while observing an endoscopic image, and performs observation or the like of a large intestine deep part.

In this way, when performing a manipulation for inserting the insertion section 10 of the endoscope 2 to the deep part of the large intestine, the surgeon selects the second tube 3B in which the second balloon 35B is provided in the second tube insertion section 31B. As a result, it is possible to firmly fix the second balloon 35B to the intestinal wall with a desired fixing force in the state in which the second balloon 35B is expanded.

As a result, it is possible to surely and smoothly perform, while preventing extension of the intestinal canal, a manipulation for pulling in the intestinal canal and straightening the large intestine and perform a large intestine test and or like.

Note that length of the first tube insertion section 31A of the first tube 3 A is AL and length of the second tube insertion section 31B of the second tube 3B is BL. BL is set shorter than AL.

The length AL is set such that the first tube insertion section 31A projects to an outside of a body by length determined in advance in a state in which the first tube insertion section 31A is inserted into the small intestine in a predetermined state. On the other hand, the length BL is set such that the second tube insertion section 31B projects to the outside of the body by length determined in advance in a state in which the second tube insertion section 31B is inserted into the large intestine in a predetermined state.

Therefore, in a manipulation for combining over-tubes, the surgeon can perform satisfactory handling irrespective of a difference of models of the over-tubes.

The third tube 3C is used in combination with an endoscope in which a not-shown hood is attached to a distal end portion of an insertion section. With the third tube 3C, before treatment, a nurse or the like attaches the hood to the insertion section of the endoscope in advance.

As a result, when performing curing and treatment by the endoscope, the surgeon can perform the curing and the treatment without inserting the insertion section of the endoscope into the tube introducing hole of the over-tube and without attaching the hood to the insertion section projected from a distal end face of the over-tube.

In this way, when outer diameter dimensions and length dimensions of the tube insertion sections configuring the over-tube are set as appropriate according to specifications of the endoscope combined with the over-tube, a size relation of diameter dimensions of balloons provided in the tube insertion sections and a size relation of axial direction lengths of the balloons are set to coincide with a size relation of outer diameters of the tube insertion sections. As a result, it is possible to obtain the endoscope system optimum for the specifications of the endoscope and excellent in convenience of use.

Whereas the size relation of the diameter dimensions of the balloons and the size relation of the axial direction lengths of the balloons are set to coincide with the size relation of the outer diameters of the tube insertion sections, a size relation of lengths of the tube insertion sections is set opposite to the size relation of the outer diameters of the tube insertion sections. As a result, in the endoscope system including the plurality of over-tubes, the surgeon can realize satisfactory handling irrespective of a difference of models of the over-tubes.

Note that the present invention is not limited to only the embodiment explained above. Various modified implementations are possible in a range not departing from the spirit of the invention.

With the endoscope system of the present invention, it is possible to realize the endoscope system of optimum specifications excellent in convenience of use by selecting the over-tube including the tube insertion section provided with the balloon corresponding to a purpose of use and combining the over-tube with the endoscope.

This application is based upon and claims priority from Japanese Patent Application No. 2014-111392 filed in Japan on May 29, 2014, disclosed contents of which are incorporated in the specification and the claims of this application.

## Claims

1. An endoscope system comprising:
an endoscope; and
a plurality of over-tubes provided with balloons to be expanded and contracted on distal end sides of tube insertion sections having flexibility, in which at least outer diameters of the tube insertion sections are different from one another, wherein
a size relation of expansion time diameters and a size relation of axial direction lengths of the balloons provided on the distal end sides of the tube insertion sections of the plurality of over-tubes are set to coincide with a size relation of outer diameters of the tube insertion sections.

2. The endoscope system according to claim 1, wherein a size relation of effective lengths of the respective tube insertion sections of the plurality of over-tubes is set to be opposite to the size relation of the outer diameters of the tube insertion sections.

3. The endoscope system according to claim 1, wherein the balloon expansion time diameters and the balloon axial direction lengths of the balloons are set such that a fixing strength in a state in which outer surfaces of the balloons press an in-vivo wall surface is a magnitude set in advance.

4. The endoscope system according to claim 3, wherein, in a configuration in which a hood is attached to a distal end of an insertion section of the endoscope, an inner diameter of tube introducing holes provided in the tube insertion sections of the over-tubes is larger than an outer diameter of the hood.
